(19) Europäisches Patentamt
European Patent Office
Office européen des brevets

(11) EP 1 541 171 B1

(12) **EUROPEAN PATENT SPECIFICATION**

(45) Date of publication and mention of the grant of the patent:
**15.09.2010 Bulletin 2010/37**

(21) Application number: **03738666.1**

(22) Date of filing: **03.07.2003**

(51) Int Cl.:
***A61P 3/00*** *(2006.01)* ***A61P 3/10*** *(2006.01)*
***A61K 38/22*** *(2006.01)*

(86) International application number:
**PCT/JP2003/008482**

(87) International publication number:
**WO 2004/004772 (15.01.2004 Gazette 2004/03)**

(54) **THERAPEUTICS FOR LOWERING THE BLOOD GLUCOSE LEVEL**

MITTEL ZUM SENKEN DES BLUTGLUKOSESPIEGELS

MEDICAMENTS POUR DIMINUER LE TAUX DE GLUCOSE SANGUIN

(84) Designated Contracting States:
**AT BE BG CH CY CZ DE DK EE ES FI FR GB GR HU IE IT LI LU MC NL PT RO SE SI SK TR**

(30) Priority: **05.07.2002 JP 2002197582**

(43) Date of publication of application:
**15.06.2005 Bulletin 2005/24**

(73) Proprietor: **CHUGAI SEIYAKU KABUSHIKI KAISHA**
**Tokyo, 115-8543 (JP)**

(72) Inventors:
- **INUI, Akio**
**Kobe-shi, Hyogo 654-0153 (JP)**
- **ASAKAWA, Akihiro**
**Kobe-shi, Hyogo 650-0017 (JP)**

(74) Representative: **Harding, Charles Thomas et al**
**D Young & Co LLP**
**120 Holborn**
**London**
**EC1N 2DY (GB)**

(56) References cited:
**WO-A-01/56592** **WO-A1-02/060472**
**WO-A2-01/87335** **WO-A2-01/92292**

- **ASAKAWA A. ET AL.: 'Antagonism of ghrelin receptor reduces food intake and body weight gain in mice' GUT vol. 52, no. 7, 01 July 2003, pages 947 - 952, XP002974155**
- **WREN A.M. ET AL.: 'The novel hypothalamic peptide ghrelin stimulates food intake and growth hormone secretion' ENDOCRINOLOGY vol. 141, no. 11, November 2000, pages 4325 - 4328, XP002951588**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 541 171 B1

Sequence Listing

<110> Chugai Seiyaku Kabushiki Kaisha
<120> Therapeutics for Diabetes Mellitus
<130> 021019
<160> 4
<210> 1
<211> 28
<212> PRT
<213> Homo sapiens
<400> 1

```
Gly Ser Ser Phe Leu Ser Pro Glu His Gln Arg Val Gln Gln Arg Lys
 1               5                  10                  15
Glu Ser Lys Lys Pro Pro Ala Lys Leu Gln Pro Arg
            20                  25
```

<210> 2
<211> 22
<212> PRT
<213> Homo sapiens
<400> 2

```
Phe Val Pro Ile Phe Thr Tyr Gly Glu Leu Gln Arg Met Gln Glu Lys
 1               5                  10                  15
Glu Arg Asn Lys Gly Gln
            20
```

<210> 3
<211> 366
<212> PRT
<213> Homo sapiens
<400> 3

```
Met Trp Asn Ala Thr Pro Ser Glu Glu Pro Gly Phe Asn Leu Thr Leu
 1               5                  10                  15
Ala Asp Leu Asp Trp Asp Ala Ser Pro Gly Asn Asp Ser Leu Gly Asp
            20                  25                  30
Glu Leu Leu Gln Leu Phe Pro Ala Pro Leu Leu Ala Gly Val Thr Ala
        35                  40                  45
Thr Cys Val Ala Leu Phe Val Val Gly Ile Ala Gly Asn Leu Leu Thr
    50                  55                  60
Met Leu Val Val Ser Arg Phe Arg Glu Leu Arg Thr Thr Thr Asn Leu
65                  70                  75                  80
Tyr Leu Ser Ser Met Ala Phe Ser Asp Leu Leu Ile Phe Leu Cys Met
                85                  90                  95
Pro Leu Asp Leu Val Arg Leu Trp Gln Tyr Arg Pro Trp Asn Phe Gly
            100                 105                 110
Asp Leu Leu Cys Lys Leu Phe Gln Phe Val Ser Glu Ser Cys Thr Tyr
        115                 120                 125
Ala Thr Val Leu Thr Ile Thr Ala Leu Ser Val Glu Arg Tyr Phe Ala
    130                 135                 140
Ile Cys Phe Pro Leu Arg Ala Lys Val Val Val Thr Lys Gly Arg Val
145                 150                 155                 160
Lys Leu Val Ile Phe Val Ile Trp Ala Val Ala Phe Cys Ser Ala Gly
                165                 170                 175
Pro Ile Phe Val Leu Val Gly Val Glu His Glu Asn Gly Thr Asp Pro
            180                 185                 190
Trp Asp Thr Asn Glu Cys Arg Pro Thr Glu Phe Ala Val Arg Ser Gly
        195                 200                 205
Leu Leu Thr Val Met Val Trp Val Ser Ser Ile Phe Phe Phe Leu Pro
    210                 215                 220
Val Phe Cys Leu Thr Val Leu Tyr Ser Leu Ile Gly Arg Lys Leu Trp

225                 230                 235                 240
Arg Arg Arg Arg Gly Asp Ala Val Val Gly Ala Ser Leu Arg Asp Gln
                245                 250                 255
Asn His Lys Gln Thr Val Lys Met Leu Ala Val Val Val Phe Ala Phe
            260                 265                 270
Ile Leu Cys Trp Leu Pro Phe His Val Gly Arg Tyr Leu Phe Ser Lys
        275                 280                 285
Ser Phe Glu Pro Gly Ser Leu Glu Ile Ala Gln Ile Ser Gln Tyr Cys
    290                 295                 300
Asn Leu Val Ser Phe Val Leu Phe Tyr Leu Ser Ala Ala Ile Asn Pro
305                 310                 315                 320
Ile Leu Tyr Asn Ile Met Ser Lys Lys Tyr Arg Val Ala Val Phe Arg
                325                 330                 335
Leu Leu Gly Phe Glu Pro Phe Ser Gln Arg Lys Leu Ser Thr Leu Lys
            340                 345                 350
Asp Glu Ser Ser Arg Ala Trp Thr Glu Ser Ser Ile Asn Thr
        355                 360                 365
```

<210> 4
<211> 412
<212> PRT
<213> Homo sapiens
<400> 4

```
Met Gly Ser Pro Trp Asn Gly Ser Asp Gly Pro Glu Gly Ala Arg Glu
 1               5                  10                  15
Pro Pro Trp Pro Ala Leu Pro Pro Cys Asp Glu Arg Arg Cys Ser Pro
            20                  25                  30
Phe Pro Leu Gly Ala Leu Val Pro Val Thr Ala Val Cys Leu Cys Leu
        35                  40                  45
Phe Val Val Gly Val Ser Gly Asn Val Val Thr Val Met Leu Ile Gly
    50                  55                  60
Arg Tyr Arg Asp Met Arg Thr Thr Thr Asn Leu Tyr Leu Gly Ser Met
65                  70                  75                  80
Ala Val Ser Asp Leu Leu Ile Leu Leu Gly Leu Pro Phe Asp Leu Tyr
                85                  90                  95
Arg Leu Trp Arg Ser Arg Pro Trp Val Phe Gly Pro Leu Leu Cys Arg
            100                 105                 110
Leu Ser Leu Tyr Val Gly Glu Gly Cys Thr Tyr Ala Thr Leu Leu His
        115                 120                 125
Met Thr Ala Leu Ser Val Glu Arg Tyr Leu Ala Ile Cys Arg Pro Leu
    130                 135                 140
Arg Ala Arg Val Leu Val Thr Arg Arg Arg Val Cys Ala Leu Ile Ala
145                 150                 155                 160
Val Leu Trp Ala Val Ala Leu Leu Ser Ala Gly Pro Phe Leu Phe Leu
                165                 170                 175
Val Gly Val Glu Gln Asp Pro Gly Ile Ser Val Val Pro Gly Leu Asn
            180                 185                 190
Gly Thr Ala Arg Ile Ala Ser Ser Pro Leu Ala Ser Ser Pro Pro Leu
        195                 200                 205
Trp Leu Ser Arg Ala Pro Pro Pro Ser Pro Pro Ser Gly Pro Glu Thr
    210                 215                 220
Ala Glu Ala Ala Ala Leu Phe Ser Arg Glu Cys Arg Pro Ser Pro Ala
225                 230                 235                 240
Gln Leu Gly Ala Leu Arg Val Met Leu Trp Val Thr Thr Ala Tyr Phe
                245                 250                 255
Phe Leu Pro Phe Leu Cys Leu Ser Ile Leu Tyr Gly Leu Ile Gly Arg
            260                 265                 270
Glu Leu Trp Ser Ser Arg Arg Pro Leu Arg Gly Pro Ala Ala Ser Gly
        275                 280                 285
Arg Glu Arg Gly His Arg Gln Thr Val Arg Val Leu Leu Val Val Val
    290                 295                 300

Leu Ala Phe Ile Ile Cys Trp Leu Pro Phe His Val Gly Arg Ile Ile
305                 310                 315                 320
Tyr Ile Asn Thr Glu Asp Ser Arg Met Met Tyr Phe Tyr Gln Tyr Phe
                325                 330                 335
Asn Ile Val Ala Leu Gln Leu Phe Tyr Leu Ser Ala Ser Ile Asn Pro
            340                 345                 350
Ile Leu Tyr Asn Leu Ile Ser Lys Lys Tyr Arg Ala Ala Ala Phe Lys
        355                 360                 365
Leu Leu Leu Ala Arg Lys Ser Arg Pro Arg Gly Phe His Arg Ser Arg
    370                 375                 380
Asp Thr Ala Gly Glu Val Ala Gly Asp Thr Gly Gly Asp Thr Val Gly
385                 390                 395                 400
Tyr Thr Glu Thr Ser Ala Asn Val Lys Thr Met Gly
                405                 410
```

**Description**

Background Art

In Japan, rapid westernization of the diet has resulted in an "age of satiation". In addition, with the spread of a mechanized civilization typified by the automobile, an increasing number of Japanese people find themselves in a state of chronic physical inactivity. Given this social background, the diabetic population in Japan has recently increased at astonishing rates to an estimated five or six million including potential patients.

Diabetes mellitus is classified into two major types, type I (insulin-dependent diabetes mellitus, or IDDM) and type II (non-insulin-dependent diabetes mellitus, or NIDDM). Type I diabetes occurs most commonly in juveniles; in this disease, islets of Langerhans in the pancreas which are responsible for insulin secretion are destroyed by viral or other infection and become totally incapable of secreting insulin. Type II diabetes occurs predominantly in middle-aged persons and in the elderly; almost all (about 95%) of the diabetics in Japan are included in this classification. People who are predisposed to diabetes on account of a tendency for insufficient insulin secretion will develop the disease as a consequence of physiological inhibition of the action of insulin, such as ingestion of excess calories, physical inactivity, obesity, stress and aging.

Diabetes mellitus is closely related to obesity and a survey shows that about two-thirds of the patients with NIDDM are either obese now or experienced obesity in the past. As a matter of fact, it has been found that the blood glucose lowering action of insulin is weak in obese people.

Obesity is increasingly prevalent and an important health problem throughout the world, particularly in developed societies. In the USA, more than half of all adults are now overweight. Allison *et al.* reported that about 280,000 US deaths in 1991 were attributable to obesity (Allison DB et al., JAMA, 282:1530-1538, 1991). The pathophysiology of obesity is known to be a sustained and excessive intake of nutrients relative to expenditure. It has been shown that a "western diet" having a high fat content is associated with an increased risk of obesity.

The key to body weight regulation is the balance between food intake and energy consumption and an imbalance between the two causes either obesity or leanness. Leptin was first discovered in 1994 and ever since it was shown to be critical to body weight regulation as an adiposity signal. There have also been discovered a lot of new peptides that are located downstream of leptin cascade and which are involved in appetite regulation. In particular, it has become clear that groups of hypothalamus-derived neuropeptides that were previously considered to have independent functions play individual roles downstream of leptin cascade and that intimate information exchange occurs among those neuropeptide groups.

Among those neuropeptides, neuropeptide Y (NPY), orexins, motilin, melanin-concentrating hormone (MCH) and agouti-related protein (AGRP) are known as appetite enhancing substances. Known as appetite suppressing substances are $\alpha$-melanocyte-stimulating hormone ($\alpha$-MSH), corticotropin-releasing factor (CRF), cocaine- and amphetamine-regulated transcript (CART), and cholesystokinin (CCK). These peptides are considered to be involved in the physiological appetite control mechanism and also to affect energy homeostasis.

Growth hormone is secreted from the anterior lobe of the hypophysis; its secretion is controlled in an ingenious way as it is stimulated by the growth hormone releasing hormone (GHRH) from the hypothalamus and suppressed by somatostatin. It has recently been shown that there is a GH secretion regulating mechanism through a different pathway than what involves GHR and somatostatin. Details of this GH secretion regulating mechanism through a different pathway have been investigated by studies on the growth hormone secretagogue (GHS) which is a synthetic compound having the activity of promoting the secretion of GH. GHS acts through a different pathway than GHRH. Briefly, GHRH activates the GHRH receptor to elevate the intracellular cAMP concentration whereas GHS activates a different receptor from the GHRH receptor to elevate the intracellular $Ca^{++}$ ion concentration as mediated by the intracellular IP3 system. The structure of GHS-R, the receptor acted upon by GHS, was identified by expression cloning in 1996 (Howard A.D. et al., Science, 273: 974-977, 1996). GHS-R is a typical G protein coupled receptor that has seven transmembrane-spanning domains and is expressed predominantly in the hypothalamus and the pituitary gland.

Further, the existence of the receptor that binds the GHS which is a synthetic compound not naturally occurring in the living organisms has prompted scientists to search for endogenous ligands that activate GHS-R upon binding. As a result, ghrelin was purified from the rat stomach and identified as a ligand specific to GHS-R (Kojima M. et al., Nature, 402: 655-660, 1999).

Ghrelin is a 28-amino acid peptide, with an n-octanoyl modification on Ser 3(3$^{rd}$ amino acid residue serine). Human ghrelin differs from rat ghrelin in two amino acid residues. Shown below are the structural formulas of rat and human ghrelin:

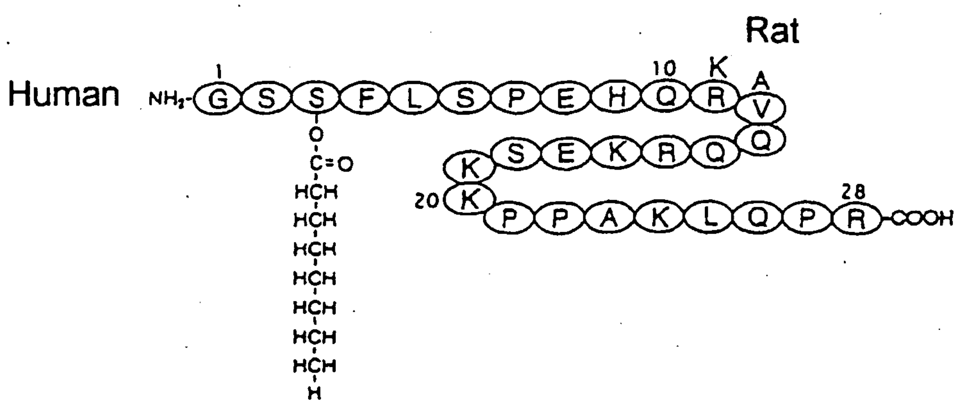


Chemically synthesized ghrelin present in an amount of the nanomolar order has the activity of elevating intracellular $Ca^{++}$ in CHO cells having GHS-R expressed therein; as well as the activity of releasing growth hormone in a primary culture of pituitary gland cells. Ghrelin also elevates growth hormone in the blood in rats in vivo. The mRNA of ghrelin is highly expressed in the stomach and ghrelin is also present in the blood. GHS-R is present in the hypothalamus, heart, lung, pancreas, small intestine and adipose tissue (*ibid.* Kojima *et al.*). In addition, ghrelin has been reported to stimulate feeding (Wren et al., Endocrinology, 141(11): 4325-4328, 2000). The present inventors previously found that ghrelin showed a marked appetite promoting action via NPY and Y1 receptor and proposed that ghrelin should be useful as a therapeutic for disease showing a symptom of hypoalimentation (PCT/JP02/00765).

DISCLOSURE OF THE INVENTION

According to the present invention there is provided a growth - hormone secretagogue receptor (GHS-R) antagonist for use in lowering the blood glucose level.

Brief Description of the Drawings

Fig. 1A shows the amino acid sequences of human ghrelin and human motilin and Fig. 1B shows the amino acid sequences of a human ghrelin receptor and a human motilin receptor, with the same amino acids being indicated by asterisks;
Fig. 2 shows the effect of IP administered ghrelin on body weight under a high-fat diet;
Fig. 3 shows the effect of IP administered ghrelin on epididymal fat mass under a high-fat diet;
Fig. 4 shows the expression of leptin, adiponectin and resistin mRNA in WAT after IP administration of ghrelin, as assessed by Northern blot analysis;
Fig. 5 shows the expression of ghrelin mRNA under a high-fat diet, as assessed by Northern blot analysis;
Fig. 6 shows the effect of IP administered [D-Lys-3]-GHRP-6 on food intake;
Fig. 7 shows the effect of ICV administered [D-Lys-3]-GHRP-6 on food intake;
Fig. 8 shows the effect of simultaneous administration of ghrelin (IP) and [D-Lys-3]-GHRP-6 (ICV) on food intake;
Fig. 9 shows the effect of IP administered [D-Lys-3]-GHRP-6 on gastric emptying rate;
Fig. 10 shows the effect of IP administered [D-Arg-1, D-Phe-5, D-Trp-7, 9, Leu-11] substance P on food intake;
Fig. 11 shows the effect of IP administered [D-Lys-3]-GHRP-6 on food intake;
Fig. 12 shows the effect of IP administered [D-Lys-3]-GHRP-6 on food intake in *ob*/*ob* mice;
Fig. 13 shows the effect of repeatedly administered [D-Lys-3]-GHRP-6 on body weight gain in *ob*/*ob* mice; and
Fig. 14 shows the effect of IP administered [D-Lys-3]-GHRP-6 on free fatty acids in *ob*/*ob* mice.

BEST MODE FOR CARRYING OUT THE INVE TION

The GHS-R antagonists which are used as active ingredients in the present invention are substances that can bind to GHS-R, thereby inhibiting the effects of the agonists. Useful GHS-R antagonists are ghrelin or ghrelin analog antagonists.
Ghrelin or ghrelin analog antagonists can typically be identified by the screening method described below (see PCT/JP02/00765).

To be specific, screening for ghrelin or ghrelin analog antagonists can be performed by, but is not limited to, administering a candidate substance to an animal in the presence or absence of ghrelin or a ghrelin analog and measuring the food intake, NPY mRNA expression, the amount of binding between NPY and its Y1 receptor, oxygen consumption, rate of gastric emptying, or the activity of vagus nerve.

Note that ghrelin is the rat ghrelin or human ghrelin or ghrelin analog that are represented by the formula I:

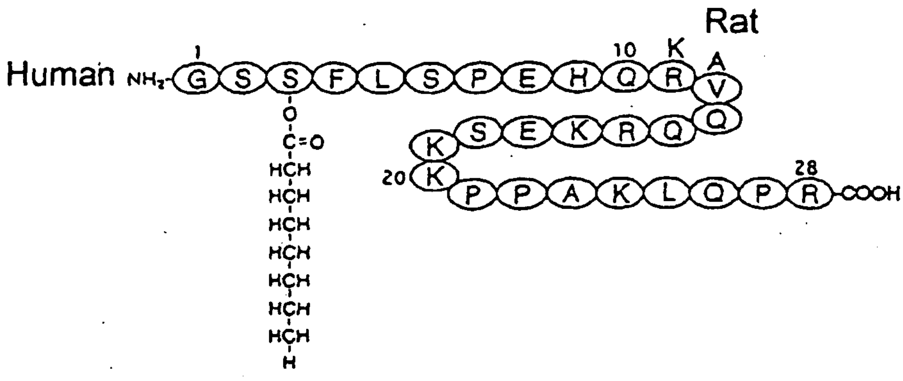

Ghrelin analogs as described herein include those which have one or more of the 28 amino acids deleted, substituted or added as long as they have the desired appetite promoting action. Also included are various derivatives of such ghrelin analogs [e.g. derivatives having peptide composing amino acids substituted (including those which have a group, say, an alkylene inserted between amino acids) and ester derivatives].

Ghrelin or ghrelin analogs may be produced by any methods and include but are not limited to those which are isolated and purified from human or rat cells, those synthesized, semi-synthesized, as well as those which are obtained by genetic engineering.

A typical example of those ghrelin analogs which have one or more of the 28 amino acids deleted, substituted or added is des-Gln14-ghrelin in which Gln 14 in ghrelin is deleted. Rat des-Gln14-ghrelin occurs due to differences in the splicing of the ghrelin gene; in the rat stomach, this analogue accounts for about a quarter of the ghrelin and provides the same intensity of growth hormone releasing activity.

Also included among ghrelin analogs are the following which are described in J. Med. Chem., 43, 4370-4376, 2000. Examples are those peptides or derivatives thereof which comprise two of the 28 amino acids in ghrelin, i.e., those at 3 and 4 positions from the N terminal (preferably the four amino acids at the N terminal) and which have the side chain at the third amino acid (Ser) from the N terminal substituted, provided that they have a growth hormone release promoting action.

The side chain at the third amino acid from the N terminal may be exemplified by acyl groups other than the octanoyl group which is the side chain in ghrelin and alky groups (the acyl and alkyl groups preferably have 6-18 carbon atoms). Specific side chains include:

$-CH_2(CH_2)_9CH_3$, $-(CO)-(CH_2)_6CH_3$, $-CO-CH=CH-CH=CH-CH=CH-CH_3$, $-CO-CH(CH_2CH_2CH_3)_2$, $-CO-(CH_2)_9CH_3$, $-CO-(CH_2)_{14}CH_3$, $-CO-(CH_2)_6CH_2Br$,
$-CO-CH(CH_2)_2CONH(CH_2)_2CH_3$, $-COPh$,

the following formulas:

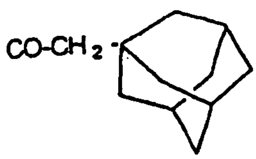 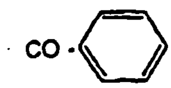

Specific examples of ghrelin analogs which comprise the amino acids at 3 and 4 positions from the N terminal and which have the side chain at the third amino acid (Ser) from the N terminal substituted include $NH_2-(CH_2)_4-CO-Ser$ (octyl)-Phe-Leu-NH-$(CH_2)_2-NH_2$, the compound reported at the 37 Peptide Forum (October 18-20, 2000).

In the present invention, [D-Lys-3]-GHRP-6 (Veeraragavan K. et al., Life Sci. 50: 1149-55, 1992) and [D-Arg-1, D-Phe-5, D-Trp-7, 9, Leu-11] substance P (Cheng, K. et al., Journal of Endocrinology, 152: 155-158, 1997) were used as GHS-R antagonists in order to verify the effects of the invention.

The therapeutics of the present invention may be administered either centrally (e.g. by intracerebroventricular route or injected into the spinal column) or peripherally. Peripheral administration is preferred. Many of the appetite regulating peptides administered peripherally do not show the same action as when they are administered centrally; however, the therapeutics of the present invention significantly lowered the blood glucose level even when they were administered peripherally. Therefore, the therapeutics of the present invention can be administered conveniently, causing less pain to the patient, thus offering far greater advantages than the conventional appetite regulating peptides.

The GHS-R antagonists can be formulated, either alone or together with pharmacologically acceptable carriers and additives, into common oral preparations and parenteral preparations by known pharmaceutical formulating procedures. For instance, they may be formulated into solution preparations (e.g. injections for intraarterial, intravenous or subcutaneous route, nasal drops and syrups), tablets, lozenges, capsules, powders, granules, ointments and suppositories. Use in drug delivery systems (as for sustained-release therapy) is also possible.

The dose of the therapeutics of the present invention varies with the age of the patient, his or her body weight, symptoms, route of administration, etc. and should be determined at a doctor's discretion. Usually, for intravenous administration, the dose ranges from about 0.1 μg to 1000 mg per kg of body weight, preferably from about 0.01 mg to 100 mg per kg of body weight, more preferably from 0.1 mg to 10 mg per kg of body weight, as calculated for the GHS-R antagonist. However, the dose is by no means limited to those ranges.

As will be described in Examples hereinafter, the present inventors measured body weight, fat mass, glucose, insulin, and gene expressions of leptin, adiponectin and resistin in white adipose tissue after repeated administrations of ghrelin under a high-fat diet. The inventors also assessed gastric ghrelin gene expression by Northern blot analysis. In addition, the inventors measured energy intake and gastric emptying after administering the GHS-R antagonists. Repeated administration of the GHS-R antagonists was continued for six days in ob/ob obese mice and their efficacy was tested.

The results were as follows: ghrelin induced appreciable adiposity and worsened glycemic control under a high-fat diet; ghrelin elevated leptin mRNA expression, and also reduced resistin mRNA expression; and gastric ghrelin mRNA expression was increased by a high-fat diet.

The GHS-R antagonists reduced energy intake in lean mice, in mice with diet-induced obesity and in ob/ob obese mice, with its mechanism of action being associated with the decrease of gastric emptying. Repeated administration of the GHS-R antagonists reduced body weight gain and improved glycemic control in ob/ob mice.

Therefore, it has been verified that ghrelin is closely related to excess body weight gain, adiposity and insulin resistance, particularly under a high-fat diet.

Discussion of the Effects of GHS-R Antagonists on Feeding

On the basis of the prediction that the GHS-R antagonists would induce a state of negative energy balance, the present inventors examined the effects of the GHS-R antagonists on feeding. The administered GHS-R antagonists reduced feeding in lean mice and in mice rendered obese by a high-fat diet. Previous reports have shown that GHS-R is present in the hypothalamus, heart, lung, pancreas, small intestine and adipose tissue. In the hypothalamus, GHS-R is located in the arcuate nucleus (ARC), where two orexigenic peptides, neuropeptide Y (NRY) and agouti-related protein (AGRP), are synthesized in neuron. In addition, nonpeptide GH secretagogues act on the hypothalamus to alter the electrical activity of ARC neurons and activate expression of the c-fos (see, for example, Lawrence et al., Endocrinology, 143: 155-162, 2002). To date, ghrelin has been reported to stimulate feeding behavior with its mechanism of action being involved in the direct activation of hypothalamic NPY and AGRP neurons in the ARC, where the blood-brain barrier is less effective (see, for example, Inui, Nat Rev Neurosci, 2: 551-560, 2001). However, an alternate pathway for ghrelin signaling from the stomach is via an ascending neural network that goes through the vagus nerve and brainstem nuclei and which ultimately reaches the hypothalamus. In the present invention, the centrally administered GHS-R antagonists abolished the stimulatory action on feeding that was induced by peripherally administered ghrelin. These results suggest that ghrelin may act through GHS-R in the brain. The present inventors also demonstrated that the peripherally administered GHS-R antagonists reduced the gastric emptying rate, which contributes to its anorexigenic action (appetite suppressing action). Considerable evidence has accumulated to indicate that gastric distention acts as a satiety signal to inhibit food intake and rapid gastric emptying is related to overeating and obesity whereas delayed gastric emptying is related to anorexia and cachexia (Inui, Mol Phychiatry, 6: 620-624, 2001). Previous studies have shown that ghrelin increases gastric emptying rate and motility through the vagal pathway (Inui, Nat Rev Neurosci, 2: 551-560, 2001).

The present invention shows that GHS-R has a role in the control of feeding behavior and that antagonism of GHS-R may be a promising approach for the treatment of obesity.

The present inventors further demonstrated that the peripherally administered GHS-R antagonists produced

an anorexigenic action and caused body weight loss and lowered blood glucose concentrations in *ob*/*ob* mice, which are known as a genetic model of obesity and diabetes mellitus with insulin resistance and rapid gastric emptying. This remarkable reduction in glucose levels, accompanied by moderate decrease in serum insulin levels, indicates the role of the GHS-R antagonists in amelioration of insulin resistance. On the other hand, it has been shown that elevations of plasma FFA induce insulin resistance through inhibition of glucose transport activity, with its mechanism of action being involved in the reduction of phosphatidylinositol 3-kinase activity. Recently, elevated circulating FFA concentration has been reported to be an independent risk factor for sudden death in middle-aged men in a long-term cohort study (Jouven et al., Circulation, 104: 756-761, 2001). In the Examples that follow, the GHS-R antagonists produced a remarkable decrease in the FFA levels of ob/ob obese mice.

In conclusion, the present inventors found that the peripherally administered GHS-R antagonists, [D-Lys-3]-GHRP-6 and [D-Arg-1, D-Phe-5, D-Trp-7, 9, Leu-11] substance P, reduced food intake in lean mice, in mice with diet-induced obesity and in ob/ob obese mice. The inventors also showed that repeated administration of [D-Lys-3]-GHRP-6 decreased body weight gain and improved glycemic control in mice.

The subject application claims priority from Japanese Patent Application 2002-197582.

The present invention is described in greater detail by reference to the following examples.

## Examples

### Test Materials and Methods

(1) Animal experiments

Male mice of the ddy strain (34-37 g, JAPAN SLC, Shizuoka, Japan) and obese (*ob*/*ob*) C57BL/6J mice (68-74 g, Shionogi Co., Ltd., Shiga, Japan) were used. They were individually housed in a regulated environment (22 ± 2°C, 55 ± 10% humidity, 12:12 hour light:dark cycle with light on at 7:00 AM). Mice received a standard diet containing 12% of total energy as fat or a high-fat diet containing 45% of total energy as fat (CLEA Japan, INC., Tokyo, Japan). Food and water were available ad lib except as otherwise indicated. All experiments were approved by Kobe University animal care committee.

(2) Test drugs

[D-Lys-3]-GHRP-6, [D-Arg-1, D-Phe-5, D-Trp-7, 9, Leu-11] substance P and rat ghrelin were purchased from BACHEM CALIFORNIA INC. (Torrance, California, USA), NEOSYSTEM (Strasbourg, France) and Peptide Institute (Osaka, Japan), respectively. Just before administration, each drug was diluted in 4 μl of artificial cerebrospinal fluid (ACSF) for intra-third cerebroventricular (ICV) administration or in 100 μl of physiological saline for intraperitoneal (IP) administration. Results are expressed as mean value ± s.e.m. Analysis of variance(ANOVA) followed by Bonferroni's t test was used to assess the differences among groups. $P$ <0.05 was considered to be statistically significant.

(3) ICV drug administration

For ICV administration, the mice were anesthetized with sodium pentobarbital (80-85 mg/kg IP) and placed in a stereotaxic instrument (SR-6, Narishige, Tokyo, Japan) seven days before the experiments. A hole was made in each skull by using a needle inserted 0.9 mm lateral to the central suture and 0.9 mm posterior to the bregma. A 24-gauge cannula (Safelet-Cas, Nipro, Osaka, Japan) beveled at one end over a distance of 3 mm was implanted into the third cerebral ventricle for ICV administration. The cannula was fixed to the skull with dental cement and capped with silicon. A 27-gauge injection insert was attached to a microsyringe by PE-20 tubing.

(4) Feeding tests

Before feeding tests, mice were food deprived for 16 hours with free access to water, except during an experiment on the effect of co-administration of [D-Lys-3]-GHRP-6 and ghrelin on food intake, in which mice were given free access to food and water. Food intake was measured by subtracting uneaten food from the initially pre-measured food at 20 minutes, 1, 2 and 4 hours after administration.

(5) RNA isolation and Northern blot analysis

RNA was isolated from the stomach and epididymal fat using the RNeasy Mini Kit (Qiagen, Tokyo, Japan). Total RNA was denatured with formaldehyde, electrophoresed in 1% agarose gel, and blotted onto a Hybond $N^+$ membrane. The membranes were hybridized with a fluorescein-labeled cDNA probe. The total integrated densities of hybridization signals were determined by densitometry (Amersham Pharmacia Biotech AB, Uppsala, Sweden). Data was normalized to glyceraldehyde 3-phosphate dehydrogenase (G3PDH) mRNA abundance and was expressed as a percentage of controls.

(6) Ghrelin gene expression

Lean mice received a standard diet containing 12% of total energy as fat or a high-fat diet containing 45% of total energy as fat for two weeks. The mice were fasted for eight hours before being killed by cervical dislocation. Immediately after that, the stomachs of the mice were removed, frozen on dry ice and stored at -80°C until preparation of Northern blots.

(7) Gastric emptying

Before the experiments on gastric emptying, mice were food deprived for 16 hours with free access to water. The fasted mice had free access to pre-weighed pellets for one hour and were then administered [D-Lys-3]-GHRP-6. The mice were deprived of food again for one or two hours after administration. Food intake was measured by weighing the uneaten pellets. Mice were killed by cervical dislocation two or three hours after the start of the experiments. Immediately after that, the stomach was exposed by laparotomy, quickly ligated at both the pylorus and cardia, then removed, and its dry content was weighed. Gastric emptying was calculated according to the following formula:

$$\text{gastric emptying (\%)} = \{1 - (\text{dry weight of food recovered from the stomach}/\text{weight of food intake})\} \times 100.$$

(8) Repeated administration

Repeated IP administrations were continued for five days in lean mice under a high-fat diet or a standard diet, and for six days in *db*/*db* obese mice under a standard diet, respectively. The mice were given the administration at 7: 00 AM and 19:00 PM. Food intake and body weight were measured daily. Serum was separated from blood obtained from the orbital sinus under ether anesthesia at the end of the experiment (eight hours after removal of food and the final IP administration). Mice were killed by cervical dislocation. Immediately after that, the epididymal fat pad mass assessed as white adipose tissue (WAT) and the gastrocnemius muscle were removed and weighed. Blood glucose was measured by the glucose oxidase method. Serum insulin and free fatty acids (FFA) were measured by enzyme immunoassay and enzymatic method (EIKEN CHEMICAL CO., LTD., Tokyo, Japan), respectively. Serum triglycerides and total cholesterol were measured by an enzymatic method (Wako Pure Chemical Industries, Ltd., Tokyo, Japan).

Example 1: The Effects of Repeated Administration of Ghrelin on Body Weight Gain and Glycemic Control Under a High-Fat Diet

As a consequence of IP administration of ghrelin twice daily for five days (single dose: 3 nmol/mouse), the body weight gain increased significantly with a concomitant increase in daily energy intake (Figs. 2 and 3, and Table 1). Fat pad mass was significantly increased by 49% and 125% compared with the physiologically saline-treated mice that were fed standard diet and high-fat diet, respectively. Skeletal muscle did not show any increase in weight. Serum cholesterol and insulin levels also increased, accompanied by a moderate increase in blood glucose concentrations. Subsequently, mRNA levels of leptin, adiponectin and resistin in WAT were assessed. Repeated administrations of ghrelin increased leptin mRNA expression, as well as reducing resistin mRNA expression in WAT (Fig. 4).

In the next place, ghrelin mRNA expression was assessed under a high-fat diet containing 45% of total energy as fat. The high-fat diet for two weeks significantly increased ghrelin gene expression in the stomach of food-deprived mice as compared with the standard diet (Fig. 5).

Table 1. Effects of ghrelin administered intraperitoneally (3 mmol/mouse every 12 hours for 5 days) on calorie intake, epididymal fat mass, gastrocnemius muscle, and blood glucose, insulin, cholesterol, triglycerides and free fatty acids concentrations in lean mice under a high-fat diet.

| | LF, Physiological saline | HF, Physiological saline | HF, Ghrelin |
|---|---|---|---|
| Calorie intake (kcal/day) | 18.83 ± 1.055 | 23.22 ± 1.329 | 25.94 ± 2.562* |
| Fat pad mass (g) | 0.533 ± 0.049 | 0.797 ± 0.095 | 1.202 ± 0.175**# |
| Skeletal muscle (g) | 0.337 ± 0.016 | 0.353 ± 0.010 | 0.340 ± 0.005 |
| Glucose (mg/dl | 142.3 ± 8.578 | 151.2 ± 14.86 | 160.5 ± 8.977 |
| Insulin (ng/ml) | 0.900 ± 0.134 | 1.183 ± 0.087 | 2.520 ± 0.945* |
| Cholesterol (mg/dl) | 144.7 ± 13.03 | 215.3 ± 19.22* | 224.8 ± 19.69** |
| Triglycerides (mg/dl) | 30.67 ± 2.860 | 27.00 ± 3.454 | 32.80 ± 7.965 |
| Free fatty acids (meq/l) | 1.467 ± 0.050 | 1.623 ± 0.100 | 1.636 ± 0.047 |

Notes: In the table, results are expressed as mean ± s.e.m. LF and HF indicate the standard diet and the high-fat diet, respectively.
*$P < 0.05$ and **$P < 0.01$ each represent the significant difference between the physiological saline-treated mice fed the standard diet and the ghrelin-treated mice fed the high-fat diet.
#$P < 0.05$ represents the significant difference between the physiological saline-treated mice fed the high-fat diet and the ghrelin-treated mice fed the high-fat diet.

Example 2: The Influence of GHS-R Antagonists on Energy Balance

The GHS-R antagonist [D-Lys-3]-GHRP-6 was IP administered to food-deprived mice. As Fig. 6 shows, [D-Lys-3]-GHRP-6 significantly reduced food intake in a dose-dependent manner.

The present inventors also investigated whether centrally administered [D-Lys-3]-GHRP-6 would have similar effects. ICV as well as IP administered [D-Lys-3]-GHRP-6 produced a potent decrease in feeding behavior (Fig. 7).

In order to evaluate the possibility that ghrelin would act through GHS-R in the brain, the inventors examined the effects of simultaneous administration of ghrelin and [D-Lys-3]-GHRP-6 on food intake. ICV administered [D-Lys-3]-GHRP-6 abolished the stimulatory effects on the feeding induced by IP administration of ghrelin (Fig. 8).

Next, the inventors examined the effect of IP administration of [D-Lys-3]-GHRP-6 on the gastric emptying rate. Peripherally administered [D-Lys-3]-GHRP-6 produced a significant decrease in the gastric emptying rate one hour after administration (Fig. 9).

The inventors also examined the effect of the other GHS-R antagonist, [D-Arg-1, D-Phe-5, D-Trp-7, 9, Leu-11] substance P (L-756,867), on feeding in food-deprived mice. Like [D-Lys-3]-GHRP-6, peripherally administered [D-Arg-1, D-Phe-5, D-Trp-7, 9, Leu-11] substance P significantly reduced food intake in a dose-dependent manner (Fig. 10).

Because repetitive vehicle administration greatly compromised the body weight gain induced by the high-fat diet (Fig. 2), the present inventors examined the acute action of [D-Lys-3]-GHRP-6 in mice with diet-induced obesity. Upon IP administration of [D-Lys-3]-GHRP-6, the food intake decreased potently, with the resulting decrease in body weight gain (Fig. 11).

To gain further insight into the therapeutic potential of GHS-R antagonists, the present inventors examined whether the IP administered [D-Lys-3]-GHRP-6 would produce an anorexigenic action in *ob*/*ob* mice. The administered [D-Lys-3]-GHRP-6 significantly reduced food intake in ob/ob mice as well as in lean mice (Fig. 12).

Finally, the inventors examined the effects of repeated administrations of [D-Lys-3]-GHRP-6 on body weight gain and glycemic control in *ob*/*ob* mice. The repeated administrations of [D-Lys-3]-GHRP-6 significantly lowered the body weight gain and blood glucose concentrations without reducing the muscle weight (Fig. 13 and Table 2). Furthermore, the treatment with [D-Lys-3]-GHRP-6 significantly reduced FFA levels in *ob*/*ob* mice by 24% compared with the physiological saline-treated *ob*/*ob* mice (Fig. 14).

Table 2. Effects of [D-Lys-3]- GHRP-6 administered intraperitoneally (20-200 mmol/mouse every 12 hours for 6 days) on food intake, epididymal fat mass, gastrocnemius muscle, and blood glucose, insulin, cholesterol, triglycerides and free fatty acids concentrations in *ob*/*ob* obese mice.

| | Physiological saline | 20 nmol | 200 nmol |
|---|---|---|---|
| Food intake (g/day) | 4.845 ± 0.160 | 4.527 ± 0.261 | 4.285 ± 0.298 |
| Fat pad mass (g) | 0.974 ± 0.066 | 0.897 ± 0.169 | 0.860 ± 0.086 |

(continued)

| | Physiological saline | 20 nmol | 200 nmol |
|---|---|---|---|
| Skeletal muscle (g) | 0.300 ± 0.012 | 0.314 ± 0.009 | 0.326 ± 0.013 |
| Glucose (mg/dl) | 234.4 ± 27.71 | 217.3 ± 27.90 | 134.9 ± 19.47* |
| Insulin (ng/ml) | 55.29 ± 11.17 | 41.61 ± 10.85 | 36.54 ± 8.695 |
| Cholesterol (mg/dl) | 257.1 ± 13.38 | 219.0 ± 11.24 | 228.4 ± 21.84 |
| Triglycerides (mg/dl) | 45.86 ± 4.378 | 38.57 ± 3.551 | 41.14 ± 5.990 |
| Free fatty acids (meq/l) | 2.164 ± 0.075 | 2.036 ± 0.121 | 1.646 ± 0.078** |

Note: *$P < 0.05$ and **$P < 0.01$ each represent the significant difference between the physiological saline-treated controls.

## Claims

1. A growth-hormone secretagogue receptor (GHS-R) antagonist for use in lowering the blood glucose level.

2. The GHS-R antagonist according to claim 1, selected from the group consisting of a ghrelin analog antagonist, [D-Lys-3]-GHRP-6 and [D-Arg-1, D-Phe-5, D-Trp-7, 9, Leu-11] substance P.

3. The GHS-R antagonist according to claim 1 or claim 2, formulated so as to be administered centrally or peripherally.

## Patentansprüche

1. Wachstumshormon-Sekretagogum-Rezeptor (GHS-R)-Antagonist zur Verwerdung in der Senkung des Blutglucosespiegels.

2. GHS-R-Antagonist nach Anspruch 1, ausgewählt aus der Gruppe bestehend aus einem Ghrelin-Analog-Antagonisten, [D-Lys-3]-GHRP-6 und [D-Arg-1, D-Phe-5, D-Trp-7, 9, Leu-11]-Stoff P.

3. GHS-R-Antagonist nach Anspruch 1 oder 2, so formuliert, um zentral oder peripher verabreicht zu werden.

## Revendications

1. Antagoniste des récepteurs des sécrétagogues de l'hormone de croissance (GHS-R) pour un usage destiné à diminuer le taux de glucose dans le sang.

2. Antagoniste de GHS-R selon la revendication 1, choisi parmi le groupe constitué d'un antagoniste d'analogue de la ghréline, de la [D-Lys-3]-GHRP-6 et de la substance P de type [D-Arg-1, D-Phe-5, D-Trp-7, 9, Leu-11].

3. Antagoniste de GHS-R selon la revendication 1 ou selon la revendication 2, formulé de sorte à pouvoir être administré au niveau central ou périphérique.

*Fig.1*

A

```
Ghrelin   1:--GSSFLSPEHQRVQQRKESKKPPAKLQPR 28
Motilin   1:FV-PIFTYGELQRMQE-KERNKGQ------ 22
                 *   * ** *  **  *
```

B

```
Ghrelin receptor (GHSR)   1:MWNATPSEEPGFNLTLADLDWDASPGNDSLGDELLQLFPAPLLAGVTATCVALFVVGIAGNLLTMLVVSRFRELRTTTNLYLSSMAFSDLLIFLCMPLDL 100
Motilin receptor          1:M--GSPWNGSDGPEGAREPPWPALP--PC-DERRCSPFPLGALVPVTAVCLCLFVVGVSGNVVTVMLIGRYRDMRTTTNLYLGSMAVSDLLILLGLPFDL 95
                                *    *              * * *            **   *  *** *  *****  **  *      * *  ********* *** ***** *  * **

Ghrelin receptor (GHSR) 101:VRLWQYRPWNFGDLLCKLFQFVSESCTYATVLTITALSVERYFAICFPLRAKVVVTKGRVKLVIFVIWAVAFCSAGPIFVLVGVEHE---------NGT- 190
Motilin receptor         96:YRLWRSRPWVFGPLLCRLSLYVGEGCTYATLLHMTALSVERYLAICRPLRARVLVTRRRVCALIAVLWAVALLSAGPFLFLVGVEQDPGISVVPGLNGTA 195
                             ***  *** ** *** *    * * ***** *  ********* *** **** * **  **    * * ****  ****   *****           ***

Ghrelin receptor (GHSR) 191:---------D-P-W-D-T-NEC--R-P-T-E----FA--VR-S-G-L--LTVMVWVSSIFFFLPVFCLTVLYSLIGRKLWRRRRGDAVVGASLRDQNHKQ 260
Motilin receptor        196:RIASSPLASSPPLWLSRAPPPSPPSGPETAEAAALFSRECRPSPAQLGALRVMLWVTTAYFFLPFLCLSILYGLIGRELWSSRRPLRGPAASGRERGHRQ 295
                                     * *            * * *   *   * *  *  * ** **  *  * ** **    ****  **  ** **** **  **      ** *   * *

Ghrelin receptor (GHSR) 261:TVKMLAVVVFAFILCWLPFHVGRYLFSKSFEPGSLEIAQISQYCNLVSFVLFYLSAAINPILYNIMSKKYRVAVFRLLGFEPFSQRKLSTLKDESSRAWT 360
Motilin receptor        296:TVRVLLVVVLAFIICWLPFHVGRIIYINT-EDSRM-MY-FYQYFNIVALQLFYLSASINPILYNLISKKYRAAAFKLLLARKSRPRGFHRSRDTAGEVAG 392
                              **  * *** *** **********          *          ** * *   ****** *******  ***** * * **        *       *

Ghrelin receptor (GHSR) 361:ESSINT--------------                                                                                 366
Motilin receptor        393:DTGGDTVGYTETSANVKTMG                                                                                 412
                                 *
```

*Fig.2*

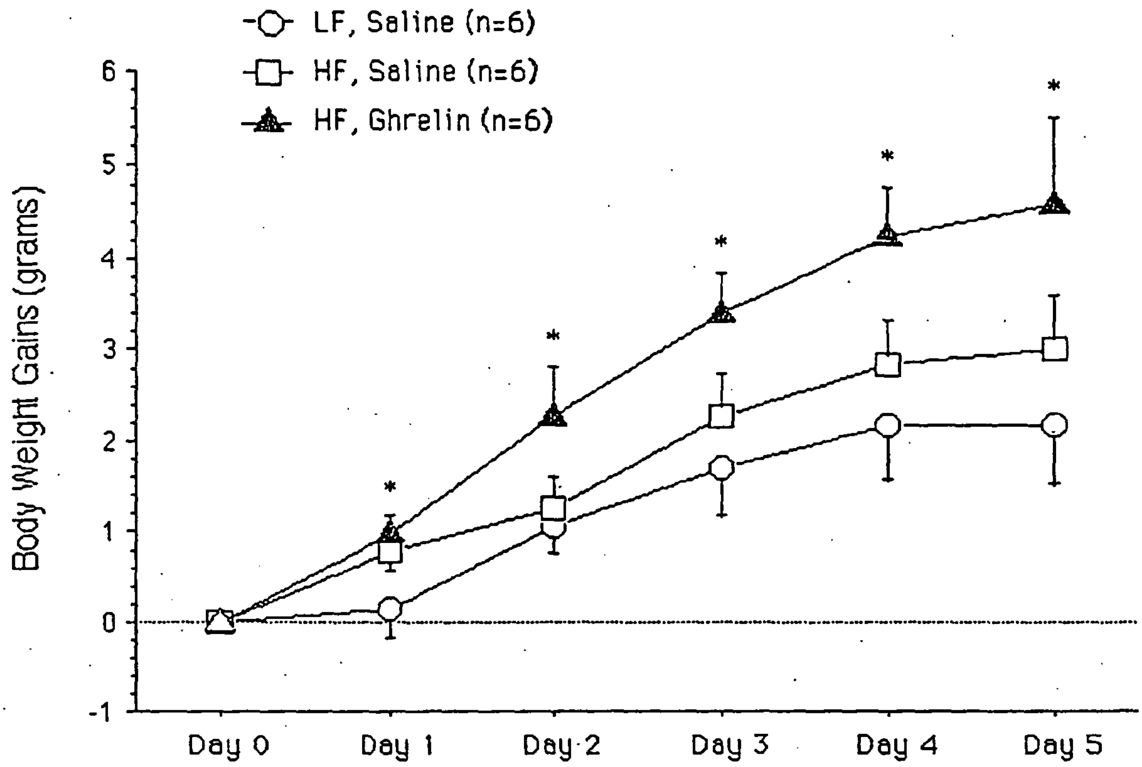
LF, Saline (n=6)
HF, Saline (n=6)
HF, Ghrelin (n=6)
Body Weight Gains (grams)
6
5
4
3
2
1
0
-1
Day 0
Day 1
Day 2
Day 3
Day 4
Day 5
*

Fig.3

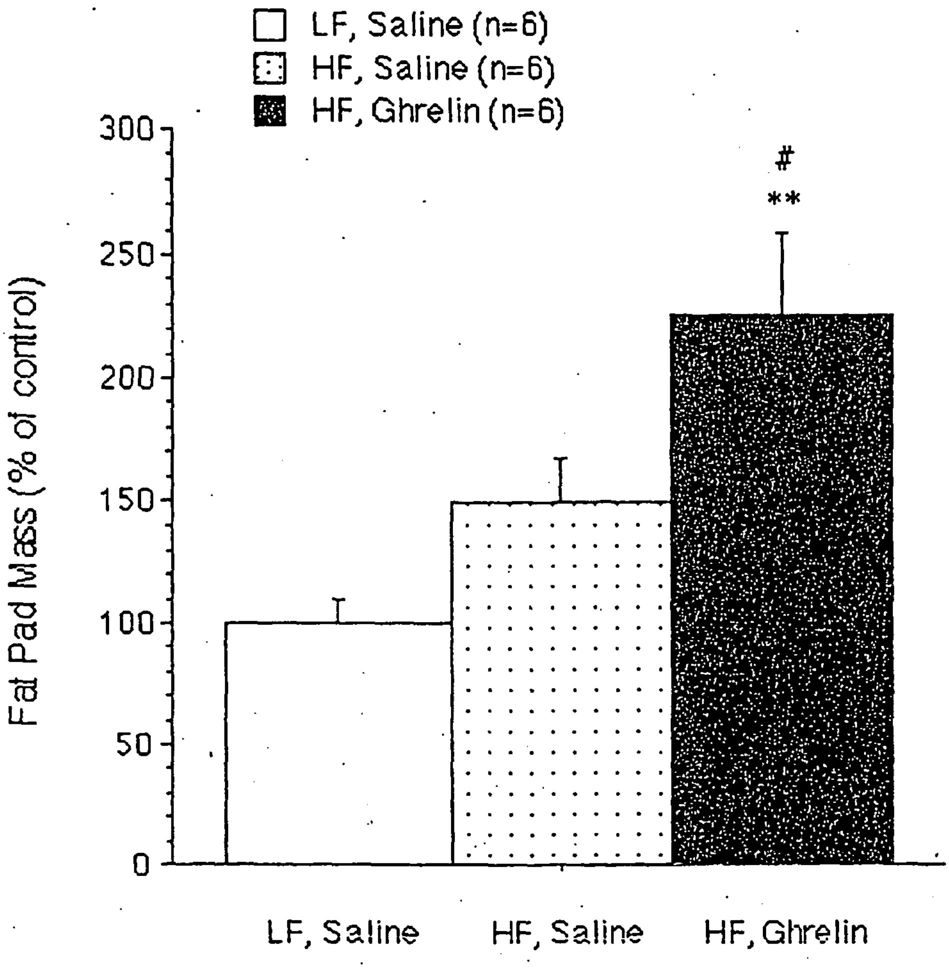
LF, Saline (n=6)
HF, Saline (n=6)
HF, Ghrelin (n=6)
Fat Pad Mass (% of control)
300
250
200
150
100
50
0
#
**
LF, Saline
HF, Saline
HF, Ghrelin

*Fig.4*

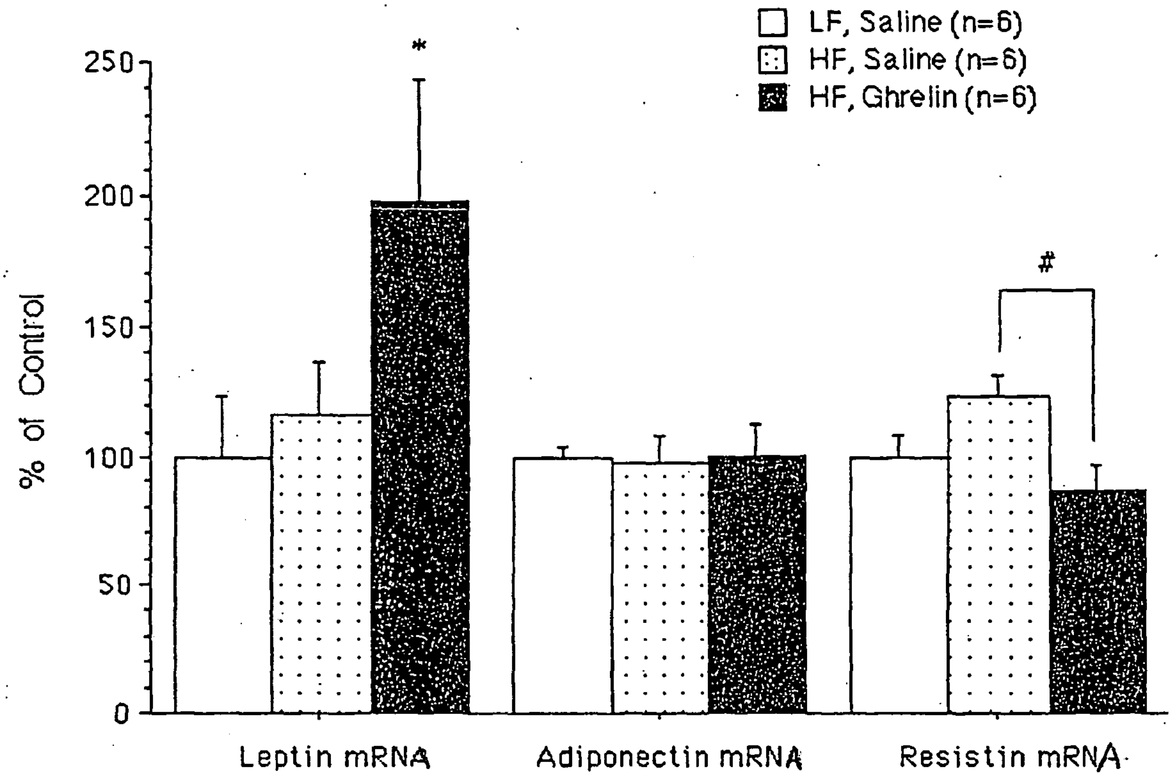
LF, Saline (n=6)
HF, Saline (n=6)
HF, Ghrelin (n=6)
% of Control
250
200
150
100
50
0
*
#
Leptin mRNA
Adiponectin mRNA
Resistin mRNA

Fig.5

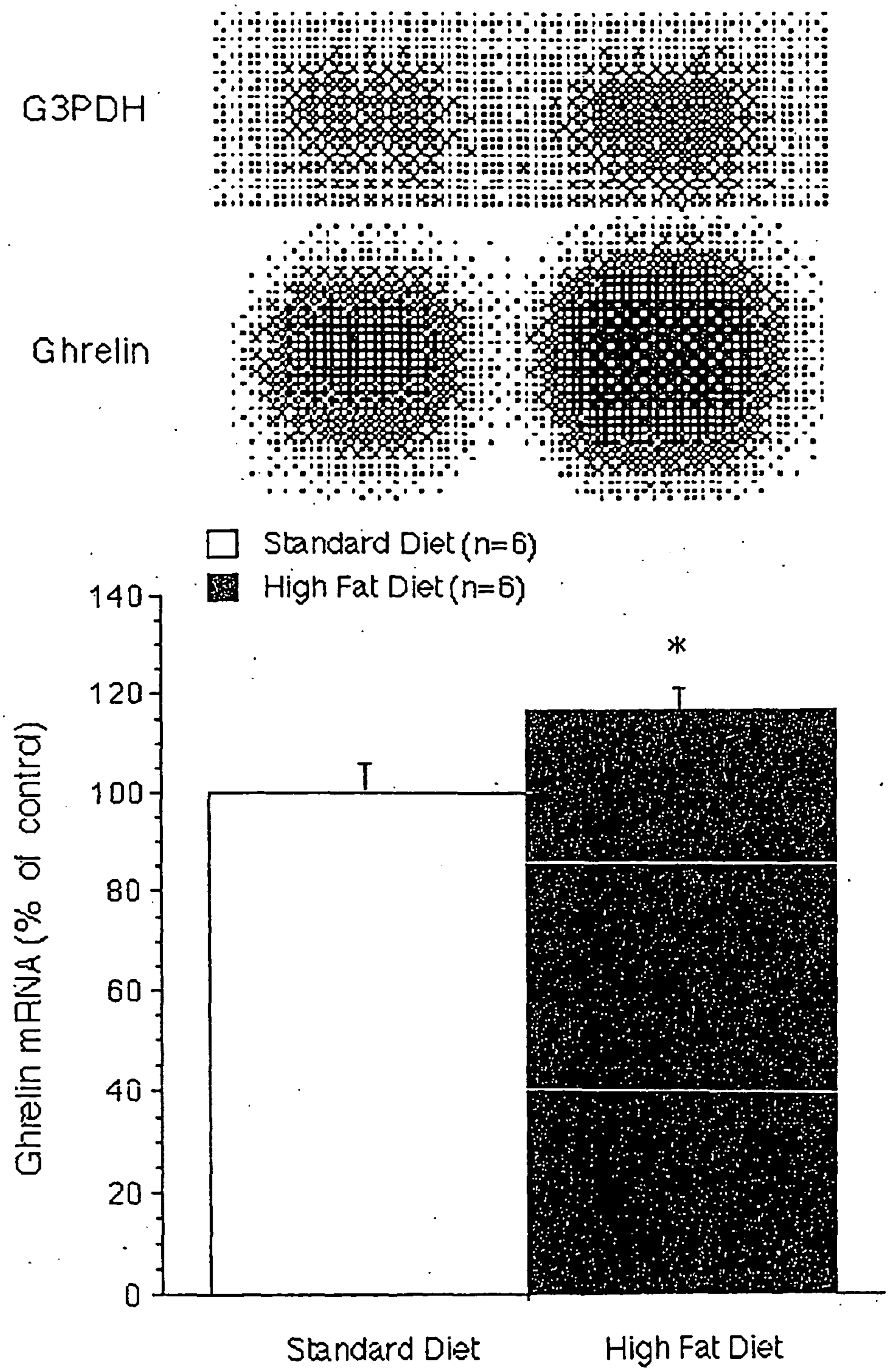
G3PDH
Ghrelin
Standard Diet (n=6)
High Fat Diet (n=6)
Ghrelin mRNA (% of control)
0
20
40
60
80
100
120
140
*
Standard Diet
High Fat Diet

*Fig.6*

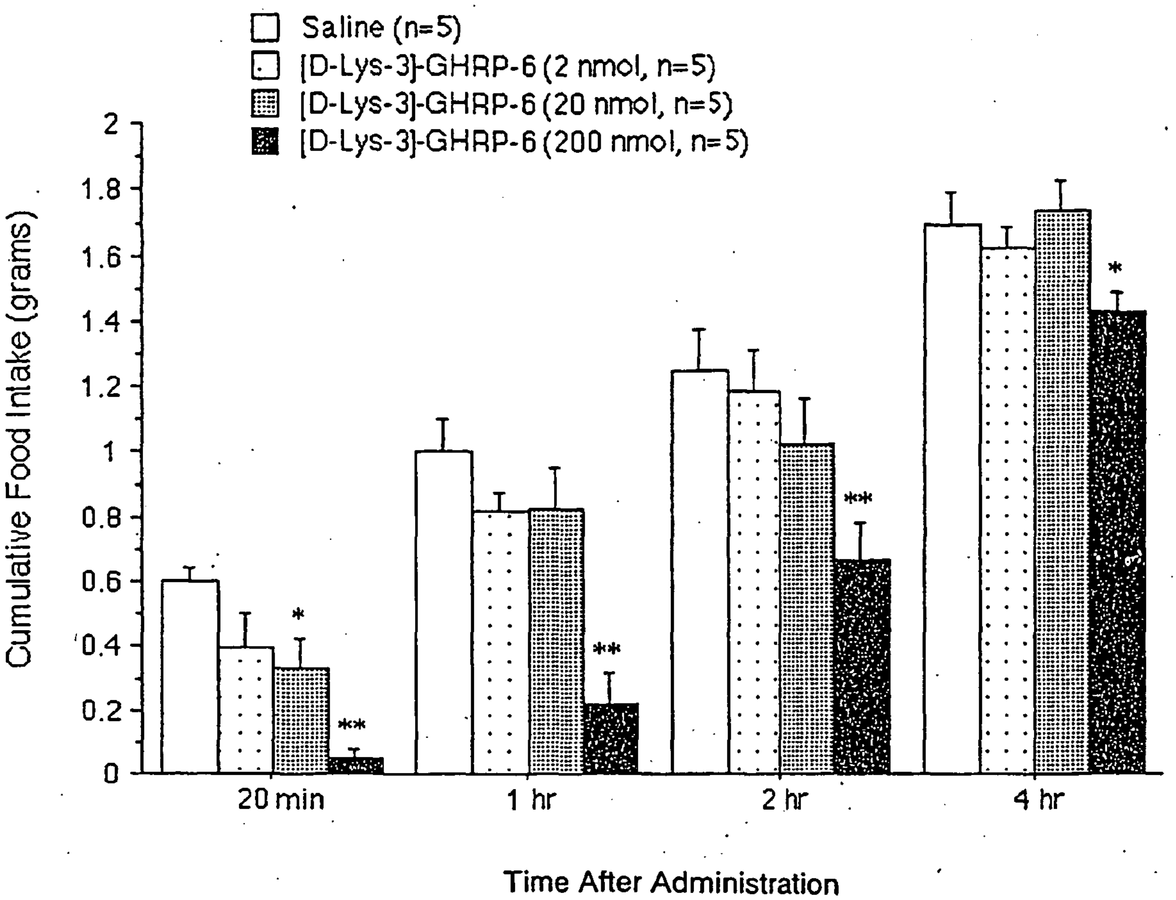
Saline (n=5)
[D-Lys-3]-GHRP-6 (2 nmol, n=5)
[D-Lys-3]-GHRP-6 (20 nmol, n=5)
[D-Lys-3]-GHRP-6 (200 nmol, n=5)
Cumulative Food Intake (grams)
2
1.8
1.6
1.4
1.2
1
0.8
0.6
0.4
0.2
0
*
**
**
**
*
20 min
1 hr
2 hr
4 hr
Time After Administration

*Fig.7*

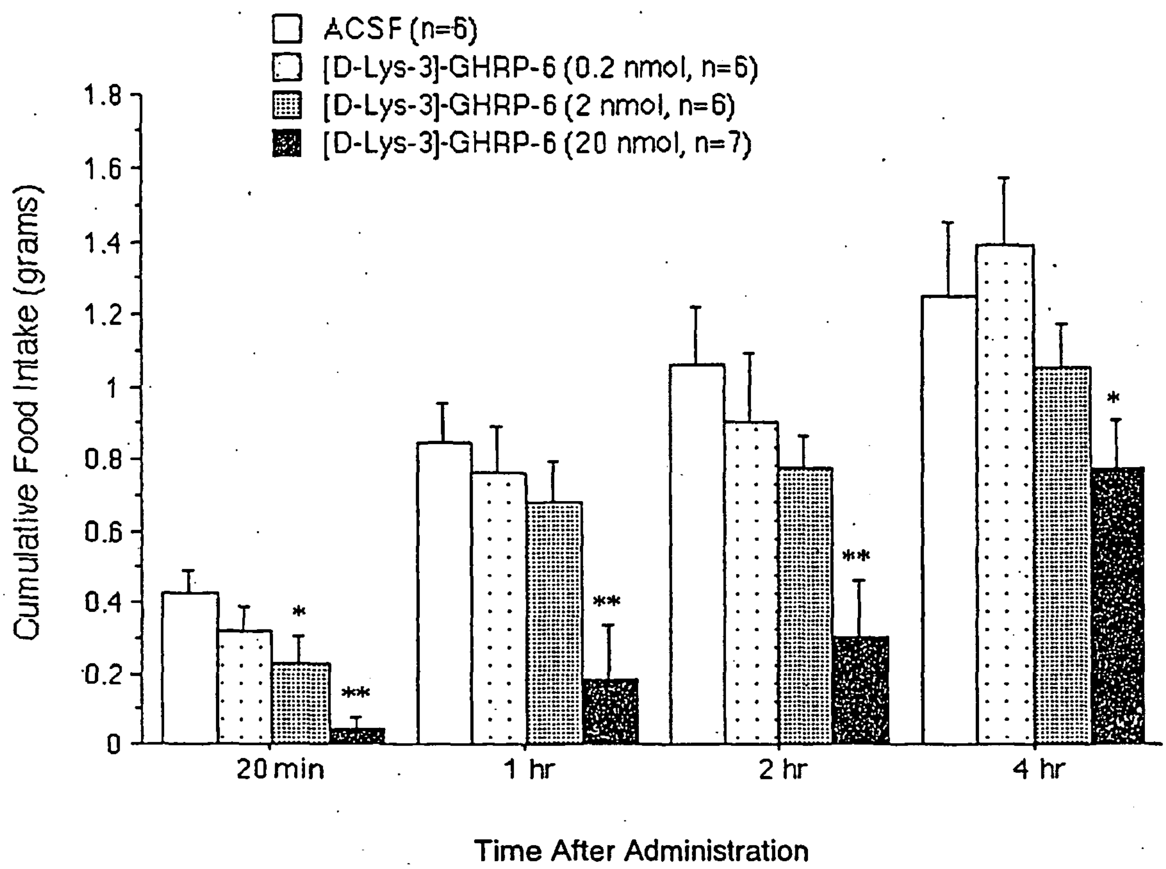
ACSF (n=6)
[D-Lys-3]-GHRP-6 (0.2 nmol, n=6)
[D-Lys-3]-GHRP-6 (2 nmol, n=6)
[D-Lys-3]-GHRP-6 (20 nmol, n=7)
Cumulative Food Intake (grams)
1.8
1.6
1.4
1.2
1
0.8
0.6
0.4
0.2
0
*
**
**
**
*
20 min
1 hr
2 hr
4 hr
Time After Administration

*Fig.8*

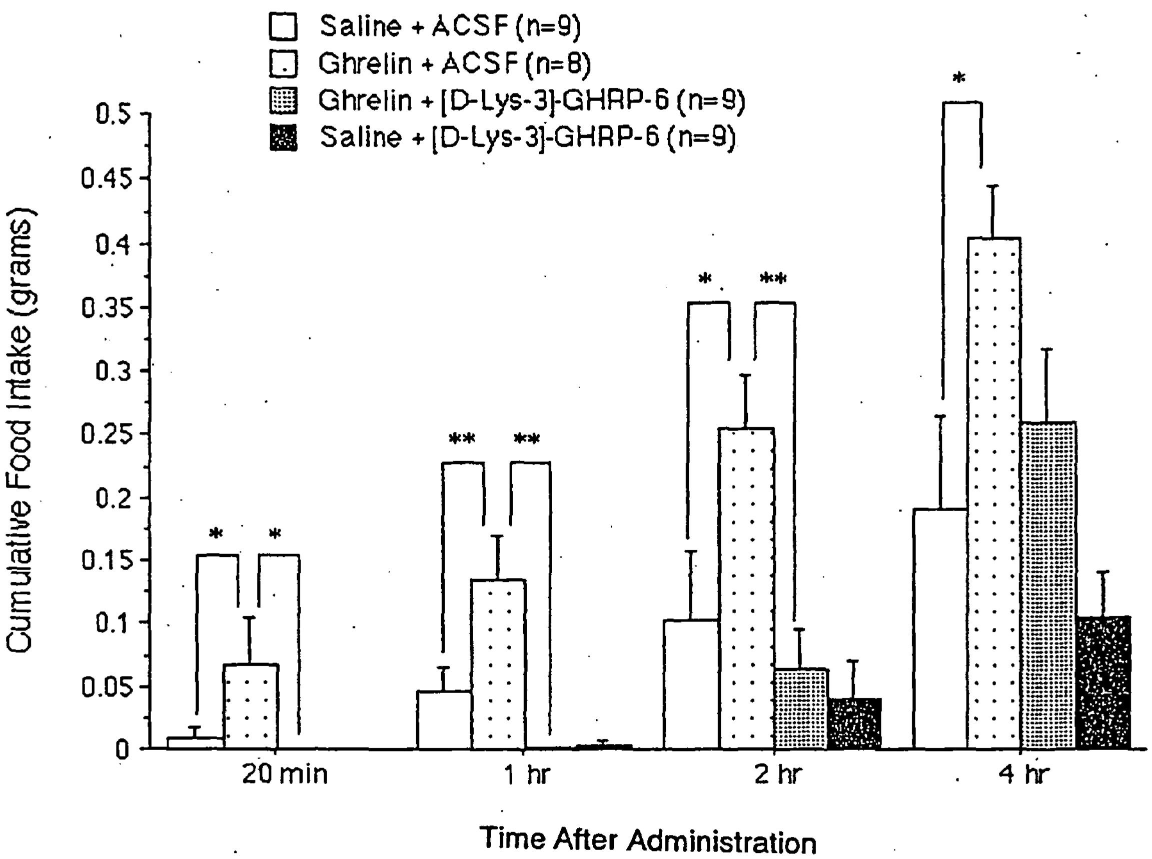
Saline + ACSF (n=9)
Ghrelin + ACSF (n=8)
Ghrelin + [D-Lys-3]-GHRP-6 (n=9)
Saline + [D-Lys-3]-GHRP-6 (n=9)
Cumulative Food Intake (grams)
0.5
0.45
0.4
0.35
0.3
0.25
0.2
0.15
0.1
0.05
0
20 min
1 hr
2 hr
4 hr
Time After Administration

*Fig.9*

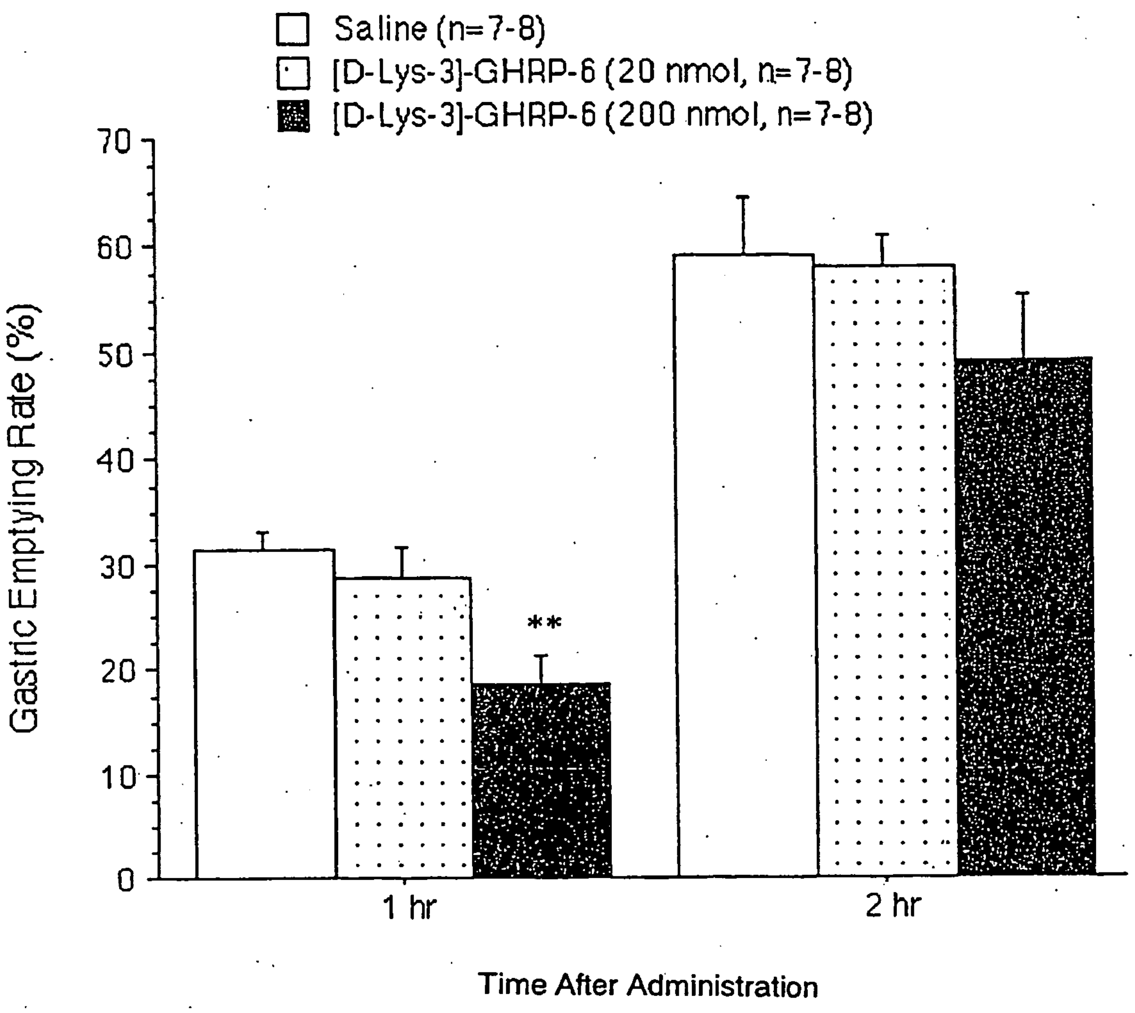
Saline (n=7-8)
[D-Lys-3]-GHRP-6 (20 nmol, n=7-8)
[D-Lys-3]-GHRP-6 (200 nmol, n=7-8)
Gastric Emptying Rate (%)
70
60
50
40
30
20
10
0
**
1 hr
2 hr
Time After Administration

Fig.10

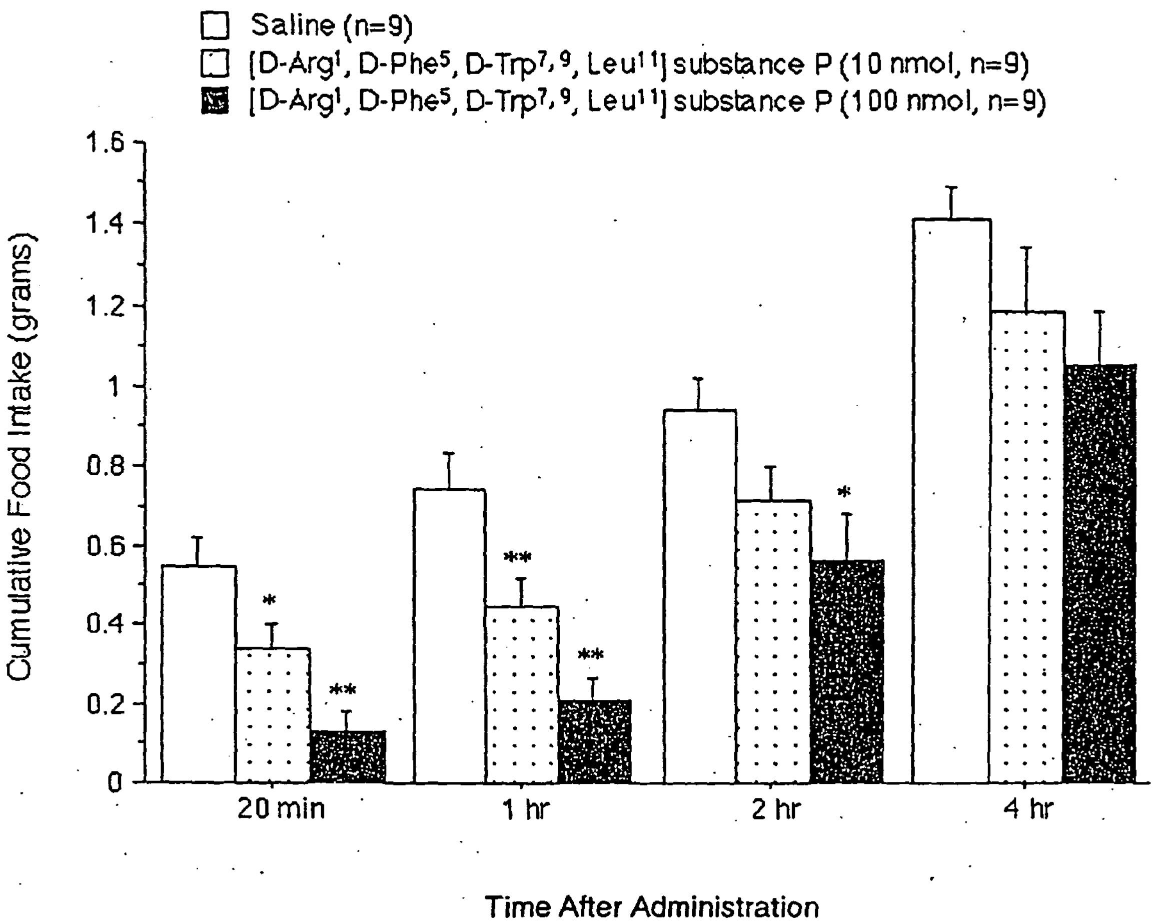
Saline (n=9)
[D-Arg1, D-Phe5, D-Trp7,9, Leu11] substance P (10 nmol, n=9)
[D-Arg1, D-Phe5, D-Trp7,9, Leu11] substance P (100 nmol, n=9)
Cumulative Food Intake (grams)
1.6
1.4
1.2
1
0.8
0.6
0.4
0.2
0
*
**
*
20 min
1 hr
2 hr
4 hr
Time After Administration

Fig. 11

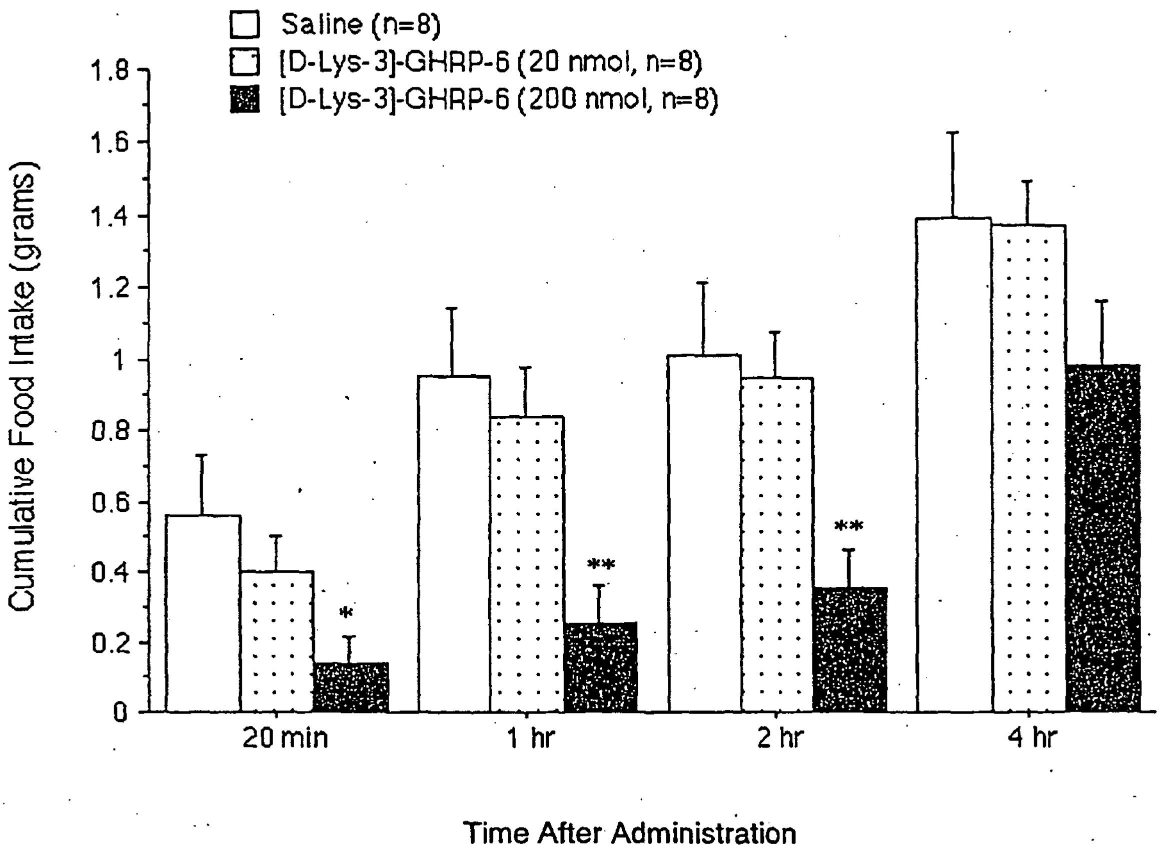
Saline (n=8)
[D-Lys-3]-GHRP-6 (20 nmol, n=8)
[D-Lys-3]-GHRP-6 (200 nmol, n=8)
Cumulative Food Intake (grams)
1.8
1.6
1.4
1.2
1
0.8
0.6
0.4
0.2
0
*
**
**
20 min
1 hr
2 hr
4 hr
Time After Administration

*Fig.12*

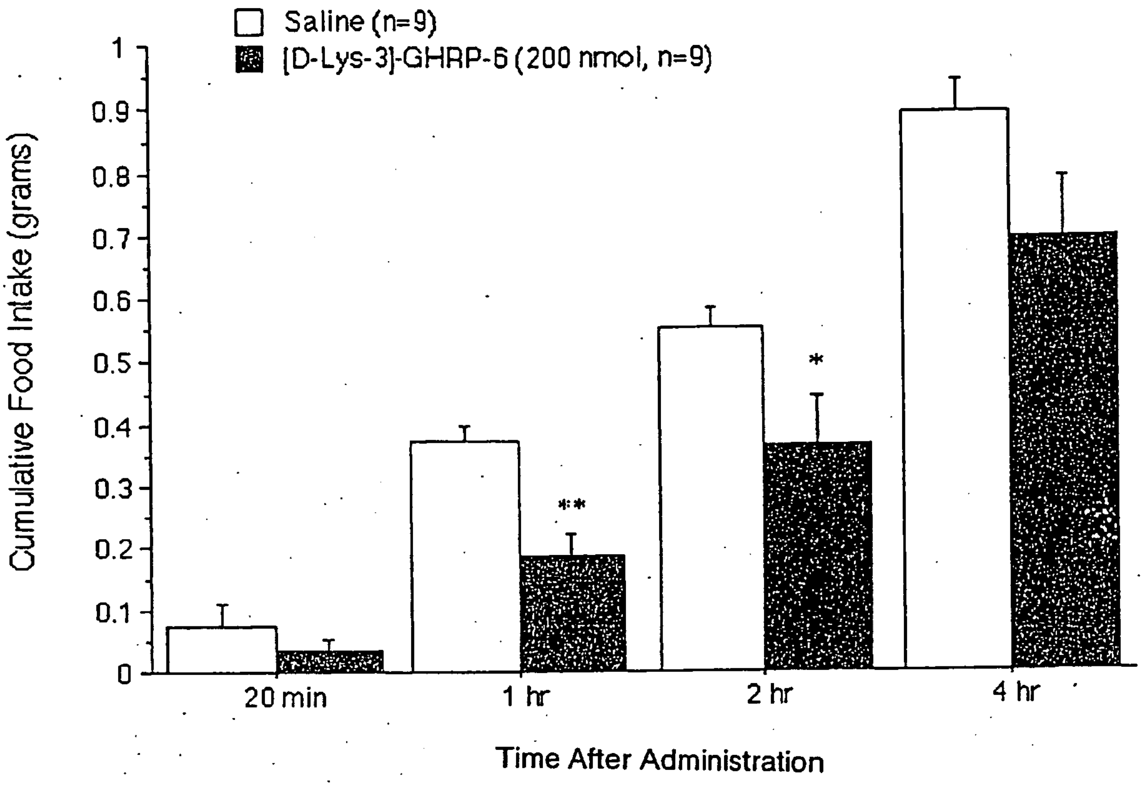
Saline (n=9)
[D-Lys-3]-GHRP-6 (200 nmol, n=9)
Cumulative Food Intake (grams)
0
0.1
0.2
0.3
0.4
0.5
0.6
0.7
0.8
0.9
1
**
*
20 min
1 hr
2 hr
4 hr
Time After Administration

Fig.13

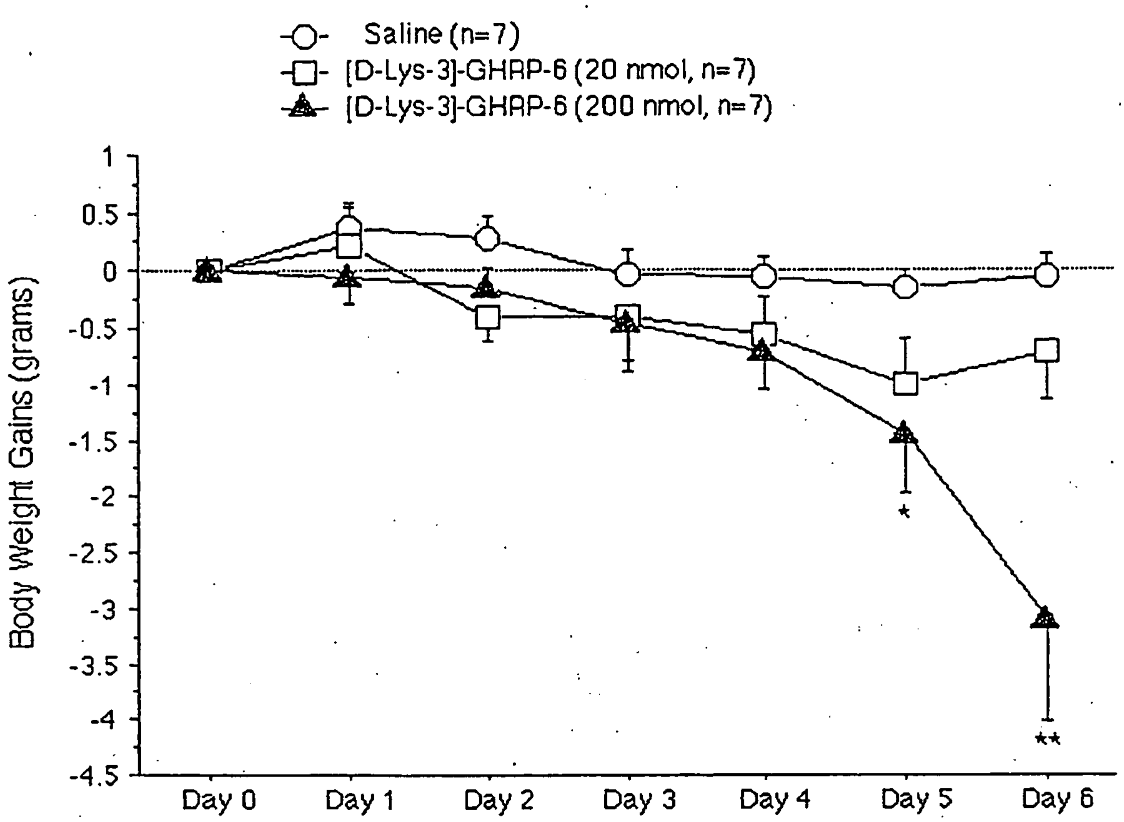
Saline (n=7)
[D-Lys-3]-GHRP-6 (20 nmol, n=7)
[D-Lys-3]-GHRP-6 (200 nmol, n=7)
Body Weight Gains (grams)
1
0.5
0
-0.5
-1
-1.5
-2
-2.5
-3
-3.5
-4
-4.5
Day 0
Day 1
Day 2
Day 3
Day 4
Day 5
Day 6
*
**

*Fig.14*

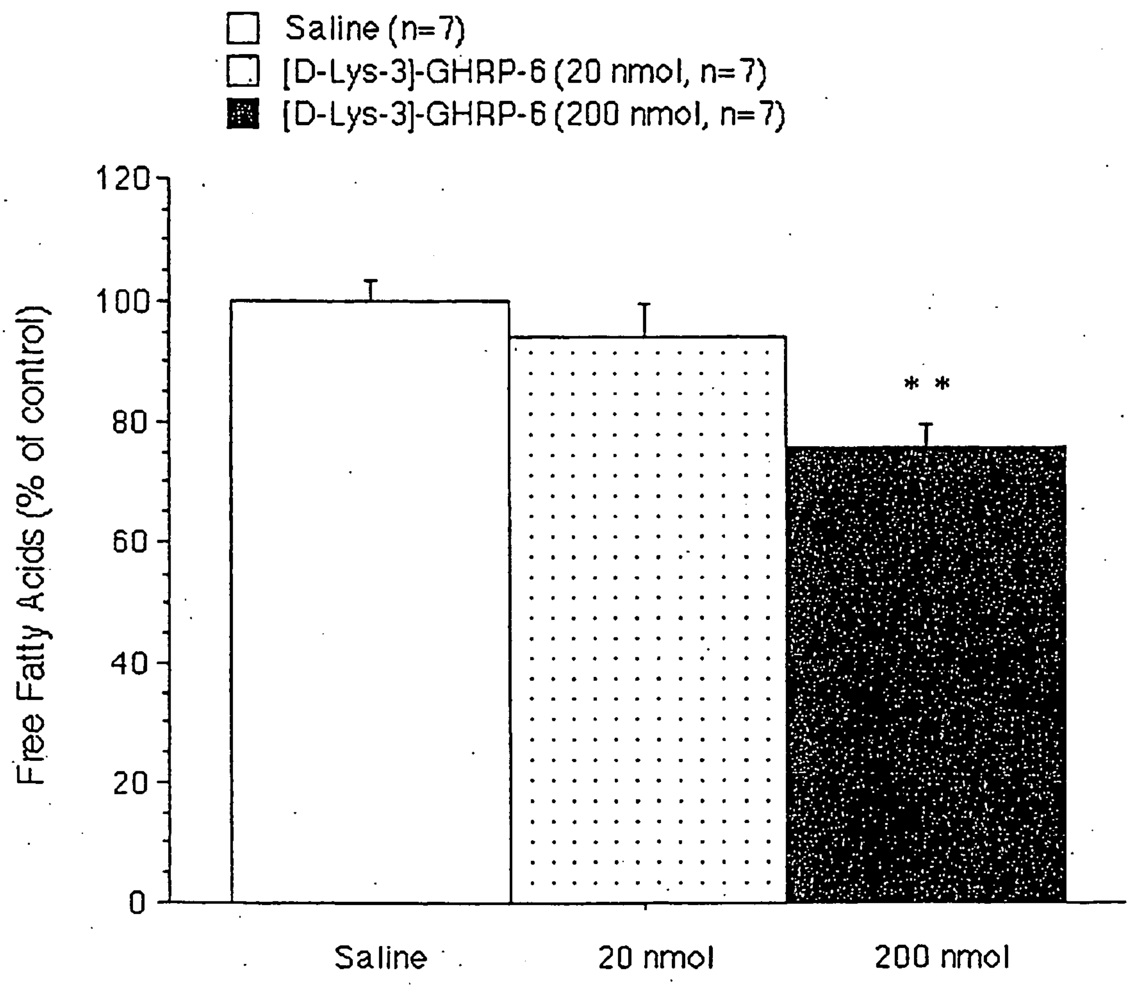

Saline (n=7)
[D-Lys-3]-GHRP-6 (20 nmol, n=7)
[D-Lys-3]-GHRP-6 (200 nmol, n=7)
Free Fatty Acids (% of control)
120
100
80
60
40
20
0
**
Saline
20 nmol
200 nmol

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000765 W
- JP 2002197582 A


### Non-patent literature cited in the description


- **Allison DB et al.** *JAMA,* 1991, vol. 282, 1530-1538
- **Howard A.D. et al.** *Science,* 1996, vol. 273, 974-977
- **Kojima M. et al.** *Nature,* 1999, vol. 402, 655-660
- **Wren et al.** *Endocrinology,* 2000, vol. 141 (11), 4325-4328
- *J. Med. Chem.,* 2000, vol. 43, 4370-4376
- **Veeraragavan K. et al.** *Life Sci.,* 1992, vol. 50, 1149-55
- **Cheng, K. et al.** *Journal of Endocrinology,* 1997, vol. 152, 155-158
- **Lawrence et al.** *Endocrinology,* 2002, vol. 143, 155-162
- **Inui.** *Nat Rev Neurosci,* 2001, vol. 2, 551-560
- **Inui.** *Mol Phychiatry,* 2001, vol. 6, 620-624
- **Jouven et al.** *Circulation,* 2001, vol. 104, 756-761